# EUROPEAN PATENT SPECIFICATION

(11) **EP 3 514 537 B1**
(45) Date of publication and mention of the grant of the patent: **16.06.2021**
(21) Application number: 18152622.9
(22) Date of filing: 19.01.2018
(51) Int. Cl.: G01N 33/50, G01N 1/28, G01N 1/31, C12N 5/071, C12N 5/09

(54) **ARTIFICIAL CONTROL TISSUE AND METHOD FOR ITS PRODUCTION**
KÜNSTLICHES KONTROLLGEWEBE UND VERFAHREN ZU DESSEN HERSTELLUNG
TISSU DE CONTRÔLE ARTIFICIEL ET SON PROCÉDÉ DE PRODUCTION

(43) Date of publication of application: 24.07.2019
(73) Proprietor: MVZ Prof. Niendorf Pathologie Hamburg-West GmbH, 22767 Hamburg (DE)
(72) Inventor: NIENDORF, Axel, 22301 Hamburg (DE)
(74) Representative: von der Lippe, Anne Lene

(56) References cited:
- BATTIFORA H: "METHODS IN LABORATORY INVESTIGATION THE MULTITUMOR (SAUSAGE) TISSUE BLOCK: NOVEL METHOD FOR IMMUNOHISTOCHEMICAL ANTIBODY TESTING", LABORATORY INVESTIGATION, NATURE PUBLISHING GROUP, THE UNITED STATES AND CANADIAN ACADEMY OF PATHOLOGY, INC, vol. 55, no. 2, 1 January 1986 (1986-01-01), pages 244-248, XP002934463, ISSN: 0023-6837
- JOHN K.C. CHAN ET AL: "Reflections on the use of controls in immunohistochemistry and proposal for application of a multitissue spring-roll control block", ANNALS OF DIAGNOSTIC PATHOLOGY, vol. 4, no. 5, 1 October 2000 (2000-10-01) , pages 329-336, XP055483768, US ISSN: 1092-9134, DOI: 10.1053/adpa.2000.17892
- SOTOLONGO B ET AL: "Gene Expression Profiling of MicroRNAs in Small-Bowel Transplantation Paraffin-Embedded Mucosal Biopsy Tissue", TRANSPLANTATION PROCEEDINGS, ELSEVIER INC, ORLANDO, FL; US, vol. 42, no. 1, 1 January 2010 (2010-01-01), pages 62-65, XP026912551, ISSN: 0041-1345, DOI: 10.1016/J.TRANSPROCEED.2009.12.018 [retrieved on 2010-02-18]
- D. A. NEUMANN ET AL: "Heart-specific autoantibodies can be eluted from the hearts of Coxsackievirus B3-infected mice", CLINICAL AND EXPERIMENTAL IMMUNOLOGY, vol. 86, no. 3, 1 December 1991 (1991-12-01), pages 405-412, XP055483675, GB ISSN: 0009-9104, DOI: 10.1111/j.1365-2249.1991.tb02945.x

## Description

### Background

The present invention relates to an artificial control tissue and methods for its production and use.

Present methods for the histomorphological (HM) analysis of cells and tissue can be used to determine the localization of an analyte in a sample and for an at least semi-quantitative determination of the analyte's concentration at specific sites. Different histochemical methods (Taatjes and Roth, Histochem Cell Biol, 2005, 124: 547-574), immunohistochemistry (IHC) methods, i.e. antibody-dependent methods (Kim et al., J Pathol Tranlat Med, 2016, 50: 411-418; Gown, Arch Pathol Lab Med, 2016, 140: 893-898) and *in situ* hybridization methods, i.e. gene probe-dependent methods, (Cassidy and Jones, Methods, 2014) can be used for a space-resolved and semi-quantitative analysis. While the analysis of the localization of an analyte has reached a high and reproducible level, there are still no truly standardized controls and reference standards for quantitative standardization of HM analysis. A number of reviews has been concerned with this lack of suitable controls in the recent past, such as Torlakovic et al. (J Clin Pathol, 2015, 68: 879-882). The authors emphasize the importance of using reliable positive and negative controls in an HM analysis. Currently, the life science community is still concerned with providing reliable standards for the qualitative analysis of a sample. The standardization of quantitative analyses is still on the level of mere guesswork, the main reason being the lack of available biological standards.

Battifora has described a method of embedding 100 or more different tissue samples in a normal-sized paraffin block, a multitumor tissue block (Battifora, LABORATORY INVESTIGATION, 1986, 55(2): 244-248.

In recent years, the need for a reliable standard has grown because HM analysis has gained importance as a prognostic tool e.g. for the diagnosis of tumors and the subsequent provision of targeted therapies to patients. Standardized quantification of certain analytes is particularly important to reduce the present inter-laboratory variance in HM analyses. Torlakovic et al. (2015) report that external proficiency testing programs have demonstrated that year after year about one-third of laboratory participants fail in providing suitable IHC tests of a high level, the reason being often the selection of inappropriate positive controls with tissues that show only high expression levels of the antigen of interest. According to Torlakovic et al. (2015), it will only be possible to determine that the proper antibody was applied, that the desired technical sensitivity and specificity is achieved, that the test is reproducible from run to run and from laboratory to laboratory and that the methodology can be transferred from literature to the clinical laboratory when standardized controls can be established.

Presently, most clinical laboratories use diagnostic tissue, such as excess tissue, as an external control for HM analysis. However, the controls do not account for the variability in tissue processing and can, at best, be similar to the analyzed tissues. Once a tissue is exhausted another reliable tissue has to be found and tested. External controls have also been developed based on peptide suspensions, cell lines, histoids constructed from several cell lines and xenografts (Torlakovic et al., 2016; Torlakovic et al., Appl Immunohistochem Mol Morphol, 2014, 22(4): 241-252). At present each laboratory has to develop its own controls. There is therefore a need in the art for standardized control tissues for HM analyses.

### Description

This need is met by the present invention with an artificial control tissue that is prepared by generating tissue fragments from different tissue samples and subsequent assembly of the fragments into an artificial control tissue. The artificial control tissue according to the invention has several advantages over prior art controls. Firstly, the artificial control tissue of the present invention reflects the high variability between different natural tissue samples due to the high number of tissue samples used for its preparation. Secondly, the artificial control tissue is reproducible with a very low inter-batch variability, thus, eliminating the need for a time-consuming validation of each new batch of artificial control tissue. Thirdly, its reproducibility and low variance ensure that the artificial control tissue is basically an inexhaustible source for the required HM controls. Fourthly, those performing the most HM analyses will generally also have access to the largest tissue sample banks, *inter alia* comprising surplus tissue which is left from former HM analyses, and can, thus, also produce a suitable amount of artificial control tissue using surplus tissue from their tissue banks.

In a first aspect, the present invention, thus, relates to a method for the manufacture of an artificial control tissue suitable for use as a control in histomorphological analysis comprising the steps of
(a) providing twenty-five or more tissue samples;
(b) shredding the tissue samples into tissue fragments;
(c) mixing the tissue fragments; and
(d) forming an artificial control tissue
wherein 50% or more of the tissue fragments comprised in the artificial control tissue have a maximal diameter of 500 µm or less.

The initial tissue samples, i.e. the tissue samples of step a), may be chosen randomly and/or be consecutive samples from a tissue storage bank.

Each tissue sample may be e.g. a sample from a tumor or from an organ of an individual. Thus, the tissue sample can be e.g. a tumor sample obtained from a benign, potentially malignant or malignant tumor. The tissue sample may also be non-tumorous tissue. The tissue sample can be selected from the group consisting of tumorous or non-tumorous tissue of the breast, colon, lung, brain, pancreas, liver, skin, prostate, ovaries, stomach, cervix, kidney, esophagus, testicles, vulva, thyroid, small intestine, thymus, larynx, nerve or uterus. Preferably, the tumor is a breast tumor (breast carcinoma). Important types of breast tumors include invasive carcinoma of no special type (NST, 8500/3), invasive lobular carcinoma (8520/3), tubular carcinoma (8211/3), cribriform carcinoma (8201/3), mucinous carcinoma (8480/3) and invasive micropapillary carcinoma (8507/3) to name but a few. The person skilled in the art is able to assess whether a tissue is tumorous or non-tumorous and to classify a tumor, if present. Suitable diagnostic methods are described e.g. in Lester and Hicks (2016, Diagnostic Pathology: Breast. 2nd edition. Salt Lake City, Utah: Amirsys Elsevier), Lakhani et al. (2012, WHO Classification of Tumours of the Breast, 4th. Lyon: IARC), Rosen et al. (2014, Rosen's Breast Pathology. 4th edition. Philadelphia: Lippincot) and Tavassoli and Eusebi (2009, Tumours of the mammary gland, AFIP Atlas of Tumour Pathology. Series 4. Washington, DC: Armed Forces Institute of Pathology).

The artificial control tissue according to the present invention consists of pieces from different tissue samples that have been shredded and artificially combined. While the shredding of the tissue samples destroys larger tissue structures, it leaves certain smaller structures, such as cell walls, parts of the extracellular matrix, membranes, cytoplasm, nuclei and a limited number of cells intact. Using the artificial control tissues, it is, therefore still possible to determine the localization of certain markers on a cellular level even though the overall structure of the original tissue has been destroyed by shredding step (b).

At the same time, the shredding of the tissue samples ensures that the generated tissue fragments are sufficiently small and preferably mixed to such a degree that each section of the artificial control tissue will show structures from a representative number of the various original tissue samples comprised in the artificial control tissue. For example, each section (e.g. of a core) of the artificial control tissue comprises tissue fragments from 10 or more different tissue samples / 0.785 mm² (e.g. in a section of a core having a diameter of about 1 mm; having a thickness of e.g. 5 µm), preferably from 20 or more / 0.785 mm², more preferably 40 or more / 0.785 mm². "Sections" can have different shapes. For example, sections can be substantially round (e.g. sections from a core) or rectangular (e.g. sections from a block of the artificial control tissue or a tissue sample).

The term "artificial", thus, refers to the artificiality of the overall, supracellular structure of the control tissue. The tissue fragments comprised in the artificial control tissue are preferably of a natural origin, i.e. were obtained from a biological organism, such as a mammal. Nevertheless, in some embodiments it is envisaged that the tissue samples have been artificially grown.

The artificial control tissue of the present invention is suitable for use as a control, e.g. in histomorphological (HM) analysis, preferably a positive control. A "control" as used herein refers to a means to verify that the processing of samples, e.g. the embedding, sectioning and the like, as well as the detection of certain markers, e.g. the staining and visual detection under the microscope has been carried out properly. Additionally, a control can be used as a standard for quantification. In that case the overall or local intensity of a detected marker in a section of the control is compared to the overall or local intensity of a detected marker in one or more sample sections.

Advantageously, the artificial control tissue disclosed herein can be reproduced in a reliable and straightforward manner once a batch is exhausted. It has been shown that the variance between batches of artificial control tissue as well as the variance within the same batch is low enough to enable a smooth transition of the histological test runs from one punch sample of a batch to the next of the same batch as well as from one batch to another. The artificial control tissue of the present invention, thus, eliminates the need for extensive searches for suitable new control tissue and, additionally, reduces the validation time for a new batch of control tissue considerably.

In a step (a) of the method of the invention, twenty-five or more tissue samples are provided.

Each tissue sample can be obtained directly from an individual, such as a test subject or a patient, or from an isolated tissue, such as tissue that has been surgically removed from an individual. Different types of tissue samples may be used, provided that they contain intact cellular structures. Preferably, the tissue sample is leftover tissue that has originally been removed from an individual for a diagnostic test and was not needed for the test *in toto.*

The tissue sample can be an embedded tissue sample, a fresh tissue sample or a frozen tissue sample. Freezing or embedding a sample can render it eligible for subsequent histological analysis, e.g. by conserving structures and simplifying sectioning of the sample. Therefore it is preferred that the tissue sample is a frozen or embedded tissue sample. Suitable embedding media are well known in the art and include, e.g. paraffin, synthetic polymers, gelatin, agar or nitrocellulose. Paraffin is particularly preferred. It is well known in the art that the type of embedding medium and also the method used to fixate and embed the tissue should be matched to the type of analysis to be carried out. For example, an electron microscopic analysis requires a different sample preparation than a conventional microscopic analysis.

To ensure a low inter artificial control tissue-variance, a number of tissue samples are provided in step (a) of the method of the invention. It has surprisingly been found that it is sufficient to pool ten or more control tissues to ensure that the resulting artificial control tissue has an acceptably low variance. Therefore, step (a) comprises providing 25 or more, more preferably 50 or more, most preferably, 100 or more tissue samples. If available, an even higher number of tissue samples can be provided in step (a), such as 250 or more tissue samples, preferably 500 or more tissue samples. In other words, in step (a) 10 to 10.000, 50 to 10.000, 100 to 10.000, 250 to 10.000, or 250 to 10.000 tissue samples are provided.

The tissue samples provided in step (a) can be pooled before or after step (b) or (c). For example, the tissue samples can be pooled first and shredded and mixed together in steps (b) and (c), the tissue samples can be shredded first and mixed subsequently in a separate step (c), or the tissue samples can be partially pooled and shredded in the pooled sub-batches in a step (b) as long as the sub-batches comprising tissue fragments are afterwards mixed in a step (c).

The tissue samples provided in step (a) can have different shapes and sizes. In certain embodiments, it can be ensured that the tissue samples provided in step (a) have approximately the same volume and/or weight (differ e.g. by not more than 40%, preferably by not more than 20%). However, it has advantageously been found that it is not necessary to match the volume or weight of the tissue samples. Variances in the size of the tissue samples are advantageously compensated for by the large tissue sample number used for generating the artificial control tissue. In embodiments in which the tissue sample volume or weight is to be matched, the skilled person will be able to devise suitable methods for ensuring that the tissue samples have approximately the same volume and/or weight. In the most straightforward version of this technique one is advised to generate 0.5 µm to 100 µm sections of each tissue sample, e.g. comprised in a paraffin block. The tissue samples may, thus, be sections having a thickness of 0.5 µm or more, preferably 1 µm or more, such as about 5 µm. In other words, the tissue samples may be sections having a thickness of from 0.5 µm to 200 µm, preferably, of from 1 µm to 100 µm. The same or approximately the same thickness and size of section will be chosen for each tissue sample. In order to harmonize the primarily implemented amount of tissue one could refine this technique by trimming the paraffin block with respect to the size of the tumor bearing part of this block in order to generate samples of equal surface.

The tissue samples may be chunks or sections. In a preferred embodiment, the tissue samples provided in step (a) are sections, e.g. sections from a paraffinated tissue. To ensure that the tissue samples have approximately the same volume and/or weight, the tissue samples may be sections from punch samples with a standardized diameter, such as a diameter of 1 mm.

It is well known in the art that tissue samples are best preserved under cryogenic conditions. Preservation of certain subcellular and cellular structures is also of advantage during the shredding of the tissue samples into tissue fragments. Moreover, it has been shown that the samples are more brittle under cryogenic conditions. The brittleness improves the processability of the samples. It is, thus, preferred that the tissue samples as well as the tissue fragments generated therefrom have a temperature of about -20°C or less during the shredding in step (b). For example, the tissue fragments as well as the tissue fragments generated therefrom may have e.g. a temperature of between -80°C and -18°C during the shredding in step (b).

In step (b) the tissue samples are shredded into tissue fragments. The shredding in step (b) can be performed using mechanical means, alternative physical means and/or chemical means. The shredding is preferably carried out mechanically. A number of mechanical methods are suitable for shredding of the tissue samples as long as it is ensured that the resulting tissue fragments have the required size for a histomorphological analysis which is described elsewhere herein. It has been found that it is preferable to use a tissue grinder, mortar or a macerator for shredding the tissue samples, most preferable to use a tissue grinder. Suitable grinders, mortars and macerators are known in the art and include standard kitchen or laboratory equipment. A suitable tissue grinder is e.g. selected from the group consisting of a porcelain tissue grinder and a glass tissue grinder, wherein porcelain tissue grinders are preferred. Nevertheless, it is also possible to use knives and other instruments using blades, balls (ball mills), roughened glass, roughened porcelain or any other physical tool to shred the tissue.

Alternatively or additionally, alternative physical means can be used. Such physical means include e.g. shredding with laser, ultrasound and water jets.

Alternatively or additionally, a chemical shredding may be used. A corresponding optional step c2) is described elsewhere herein. Chemical methods which are suitable for disintegrating tissue samples are known in the art. The chemical shredding may e.g. be a biochemical shredding. Suitable chemical, e.g. biochemical, shredding includes shredding by enzymatic digestion (compare optional step c2) described elsewhere herein).

Importantly, the shredding of the tissue samples is random. This means that the shredding does not include selecting particular sites in the samples for separating individual fragments from the sample. Meanwhile, this does not exclude that the tissue samples used in step (a) may have been separated (e.g. by cutting) from larger tissue specimen in a selective manner. Due to the unspecific shredding procedure in step (b), the resulting tissue fragments do not all have the same size and shape. Sizes and shapes of the tissue fragments comprised in the artificial control tissue vary considerably. For example, the size of the tissue fragments comprised in an artificial control tissue differs at least by a factor of 10 or more, preferably 25 or more. While a biochemical shredding using enzymatic digestion cannot be considered to be random on a molecular level, it is random on the level of cellular and connective tissue structures.

Preferably, the tissue samples are shredded into pieces of similar size and/or shape. However, a variance in the size and shape of the tissue fragments is allowed and even desirable as this reflects the structural inter- and intra-sample variance. While the grinding of a tissue sample can result in a relatively high variance in the size and shape of the resulting tissue fragments, it is desirable that the intra-sample size and shape variance is substantially the same (i.e. comparable) in all shredded tissue samples.

The tissue fragments obtained from step (b) have a size that renders the resulting artificial control tissue suitable for a histomorphological analysis and ensures that a single section comprises tissue fragments from several different original tissue samples. Therefore, the size of the fragments will be considerably smaller than a section for HM analysis (having a surface area of e.g. 1 cm² in sections cut directly from a tissue block or a diameter of e.g. 1 mm in circular sections cut from a tissue core). This way, it is ensured that the control section in the HM analysis comprises fragments from more than one, preferably from at least 10, more preferably at least 30 different tissue samples. A standard control section cut from a core for an HM analysis may have a diameter of about 1 mm and a thickness of about 5 µm.

At the same time, the size of the fragments is large enough to comprise single cells or even small cell assemblies and fragments of connective tissue, if applicable (i.e. present in the initial tissue samples). This way, the artificial control tissue can be used for an analysis of the localization of a marker. The tissue fragments can be irregularly shaped. While it has been shown that one shredding step, e.g. a mechanical shredding step, is sufficient to generate tissue fragments of the desired size, it is possible to include a further shredding step, e.g. a chemical shredding step, such as optional step c2) described elsewhere herein, to further loosen certain bonds within the tissue fragments.

In the invention, 50% or more of the tissue fragments comprised in the artificial control tissue have a maximal diameter of 500 µm or less.

The tissue fragments comprised in the disclosed artificial control tissue will usually have a maximal diameter of less than 5 mm. This means that the tissue fragments are in no direction longer than 5 mm. In other words, the tissue fragments comprised in the artificial control tissue preferably have a maximum diameter of 5 mm or less, preferably 1 mm or less, more preferably 500 µm or less. Thus, in one aspect, the tissue fragments comprised in the artificial control tissue have a maximum diameter of 1 mm or less. The diameter of the fragments can be measured in a section having a diameter of from 0.6 mm to 2 cm (e.g. in a 1 mm section from a core or a section of a tissue block having a surface area of about 1 cm²). The section can have a thickness of e.g. about 5 µm.

Alternatively, the maximum diameter of the fragments can be measured under a suitable microscope after shredding and before forming the artificial control tissue
Fragments comprising predominantly extracellular matter (connective tissue) have been found to be larger on average than fragments comprising predominantly cellular matter. Therefore, it is preferred that fragments comprising extracellular matter (connective tissue) to an extent of 70% or more ("extracellular matter-based tissue fragments") have a maximum diameter of 5 mm or less, preferably 1 mm or less, more preferably 500 µm or less. In other words, extracellular matter-based tissue fragments have preferably a maximum diameter of from 5 µm to 5 mm, preferably 5 µm to 1 mm, more preferably 5 µm to 500 µm.

Fragments comprising cellular components to an extent of 50% or more ("cell-based tissue fragments") are smaller on average than extracellular matter-based tissue fragments. It was found that many cell-based tissue fragments comprised aggregates of 10 to 100 cells, having a maximal diameter of 20 to 200 µm. Cell-based tissue fragments, thus, preferably have a maximum diameter of 500 µm or less, preferably 200 µm or less. In other words, cell-based tissue fragments preferably have a maximum diameter of from 20 µm to 500 µm, more preferably of from 20 µm to 200 µm.

It is preferred that each section having a thickness of 5 µm and a diameter of 1 mm (e.g. a section from a core) comprises at least 2, preferably at least 5, more preferably at least 10, cell-based tissue fragments, e.g. cell-based tissue fragmentscomprising from 10 to 100 cells.

The aforementioned size restrictions apply preferably to 50% or more of all tissue fragments in the disclosed artificial control tissue, more preferably 70% or more, most preferably 90% or more. For example, in one aspect, 50% or more of the tissue fragments comprised in the artificial control tissue have a maximum diameter of 1 mm or less. Alternatively or additionally, e.g., 50% or more of the extracellular matter-based tissue fragments have a diameter of 1 mm or less, and/or 50% or more of the cell-based tissue fragments have a diameter of 200 µm or less

In the invention, 50% or more of the tissue fragments comprised in the artificial control tissue have a maximal diameter of 500 µm or less.

Usually one can see intact nuclei, fragments of cytoplasm including entire organelles, as well as membrane fragments in a section of the artificial control tissue. A usually quite dominant part of the artificial control tissue are fairly large fragments of fibers which contribute to the connective tissue within the tumour stroma.

According to step (c), the tissue fragments resulting from step (b) are mixed. Steps (b) and (c) may be carried out simultaneously or successively. For example, shredding the tissue samples in a macerator will at the same time provide for a sufficient mixing of the resulting tissue fragments. Alternatively or additionally, mixing can also occur in dewaxing, rehydration and washing steps described herein.

The method according to the invention may comprise an additional step (c1), wherein the tissue fragments are freed from their embedding medium, e.g. dewaxed (deparaffinated). At the same time, the tissue fragments may be rehydrated. Standard methods known in the art may be used.

Further, the method of the invention may comprise an optional step (c2), wherein the tissue fragments are digested enzymatically. This means that a proteolytic enzyme is added to the tissue fragments. Suitable enzymes for an enzymatic digestion are known in the art and include, e.g., Trypsin. After the incubation with the proteolytic enzyme, the tissue fragments are washed to remove residual enzyme. Preferably, step (c2) is carried out after step (c1). As mentioned elsewhere herein, optional step (c2) amounts to a chemical (biochemical) shredding in the sense of the invention.

After the tissue fragments have been thoroughly mixed, an artificial control tissue is formed in step (d). Step (d) can be used to shape the mass of mixed tissue fragments into a shape suitable for further processing, e.g. into a block shape. The skilled person is aware of block sizes and shapes which are suitable for further processing for histomorphological analyses.

Step (d) may comprise compacting the mixed tissue fragments, e.g. by centrifugation. Compacting of the mixed tissue fragments reduces the free spaces between the tissue fragments in the artificial control tissue and, thus, increases the density of the tissue. In consequence, the resulting control sections used in histomorphological analysis have a high density of information. Suitable centrifugation conditions are e.g. centrifugation at 10.000 rcf to 40.000 rcf, e.g. at 20.800 rcf. Suitable centrifuges are well known in the art and include *inter alia* centrifuge 5417 R (Eppendorf) and the like. A suitable centrifugation step in the aforementioned centrifuges may be performed e.g. at about 14 000 rpm.

Step (d) can also comprise drying of the pellet, such as drying for 12 h or more, e.g. about 24 h.

Step (d) may further comprise embedding the mixed tissue fragments in an embedding medium, such as paraffin. Embedding the tissue fragments may be carried out after compacting of the tissue fragments. The density of the resulting artificial control tissue will then not be substantially changed (e.g. increased) by the embedding procedure. Suitable embedding media include paraffin, synthetic polymers, gelatin, agar and nitrocellulose. Methods for embedding tissue samples in these media are well known in the art. After hardening of the embedding material, the tissue fragments will be enclosed in and protected by the embedding material.

After formation of an artificial control tissue, the tissue can be used in histomorphological analysis as a control tissue. For this purpose, sections can be cut directly from the artificial control tissue or from a core, such as a punch sample, that has been obtained from the artificial control tissue. Alternatively, the artificial control tissue or a core thereof may be directly incorporated into a tissue microarray (TMA) or tissue block by standard methods in the art. Sections for histomorphological analyses are then cut directly from the TMA or the tissue block. Incorporation of the artificial control tissue into a TMA has the advantage that the control and the further samples comprised in the TMA are cut and subsequently treated in an identical manner. Thereby the comparability of samples and control is ensured. The sections may e.g. be made with a microtome and subsequently mounted on a glass or plastic slide. The further handling of the sections is routine in the art of histomorphological analyses.

While the tissue samples used in step (a) can advantageously be selected randomly, it can, under certain, special conditions, be useful to make a pre-selection of the samples and choose only samples of a certain group which share at least one common feature. The common feature may be a marker, such as a marker protein, nucleic acid, carbohydrate or the like. This way, the artificial control tissue can be selectively enriched in the common feature. For example, it is known that ethnic groups show different expressions for some markers. In case these are crucial for the diagnosis to be made, it can be useful to use tissue samples from individuals from the same ethnic group. In a second scenario, certain markers are expressed on a very low level in the majority of the population, while the markers are overexpressed in a small group of patients. In the unlikely event that these markers cannot be detected adequately in the artificial control tissue, it can be useful to use tissue samples which show a high expression level for a certain marker in step (a) to generate a suitable positive control for the labeling of this marker. Therefore, in particular embodiments of the invention, the tissue samples provided in step (a) preferably share at least one common feature distinguishing the samples from other samples of the same type.

For example, the tissue samples can be from the same type of organ, from the same type of tumor and/or from patients with the same ethnical and/or geographic origin. In a preferred embodiment, the tissue samples are from the same ethnical and/or geographic origin. Thereby, certain markers, which are underrepresented in other ethnical groups, can be enriched in the artificial control tissues of the present invention.

A marker enrichment can also be achieved by pooling samples which have been obtained from a tissue type or tumor type that is enriched in the respective marker. A "marker" in the sense of the present invention is a molecule (e.g. a protein or complex, gene, RNA or other physical or biochemical properties) that is detectable in diagnostic processes in which sections of the artificial control tissue serve as a control. A marker can be, e.g. a tumor marker, such as HER2, a marker for a particular cellular structure, such as actin, or the like. Preferably, the tissue samples are enriched in a diagnostic marker, i.e. a marker that can be used in the diagnosis of a disease or condition, in comparison with the standard concentration of the marker in the population. In other words, the samples provided in step (a) can express the same molecule differentially in comparison with the average localization and/or the average amount of the molecule in the same type of tissue sample.

A "differential" expression refers to an expression that differs from the average expression in the population in terms of its expression level or in terms of the expression pattern in and/or outside of cells, i.e. in the localization. For example, certain tumor markers are overexpressed in tumorous tissue. Therefore, the tissue samples used in step (a) may e.g. be enriched in a tumor marker in comparison with the average expression levels in the population.

In a second aspect, the invention pertains to artificial control tissue suitable for use as a control in histomorphological analysis and manufactured according to the method of the invention. In a related aspect, the invention relates to artificial control tissue suitable for use as a control in histomorphological analysis and comprising fragments of 25 or more tissue samples, wherein 50% or more of the tissue fragments comprised in the artificial control tissue have a maximal diameter of 500 µm or less. In a preferred aspect, the artificial control tissue comprises fragments of 50 or more, most preferably 100 or more tissue samples. The artificial control tissue can be e.g. a block, core (punch sample) or section. Usually, the artificial control tissue will initially be a block.

In a third aspect, the invention relates to a core or section from an artificial control tissue of the invention and/or manufactured according to a method of the invention. A "core" is part of the artificial control tissue. As mentioned above, the artificial control tissue will initially be a block. For further use, transport or storage, the artificial control tissue can be cut into parts which are adapted to the intended use. A "core" may, e.g. be a punch sample, i.e. a sample obtained from the artificial control tissue by punching, or a cut sample, i.e. a sample obtained from the artificial control tissue by cutting. A core may have the form of a cylinder.

As the skilled person understands, a "section" (histological section) is a part that has been cut from an artificial control tissue (block) or a core (thereof). Accordingly, the section according to the invention can e.g. be a section cut from the artificial control tissue or a section cut from a core of the artificial control tissue. A section cut from the artificial control tissue can have a surface area of e.g. about 1 cm². Depending on the diameter of the punch needle, a section of a core can have a diameter of from 0.1 to 10 mm. Thus the sections (e.g. core sections) of the invention have preferably a diameter of from 0.1 to 10 mm, more preferably 0.6 to 6 mm, such as about 1000 µm. A section can have a thickness of about 0.5 µm to 200 µm, preferably 0.5 µm to 100 µm.

In a fourth aspect, the invention relates to a tissue microarray or other tissue block comprising at least one core of an artificial control tissue according to the invention or manufactured according to a method of the invention. Methods for the preparation of tissue microarrays and other types of tissue blocks are well known in the art.

In a fifth aspect, the invention relates to an object slide (object carrier) comprising a section of an artificial control tissue according to the invention or manufactured according to a method of the invention; a section of a core according to the invention; or a section of a tissue microarray or tissue block according to the invention as an *on-slide* control. Object slides comprising the sections as an *on-slide* control can be prepared and stored for future use. For example, sections of tissue samples to be analyzed may applied to the object slide at a later stage. Methods for the application of the sections according to the invention to object slides are well known in the art.

In a sixth aspect, the invention relates to an *in vitro* diagnostic method comprising
(i) providing at least one tissue section of an artificial control tissue according to the invention or manufactured according to a method of the invention;
(ii) providing at least one tissue section of a tissue sample from an individual;
(iii) detecting at least one structure, complex and/or molecule in the tissue samples of (a) and (b); and
(iv) comparing the localization and/or the expression level of the structure, complex and/or molecule in the at least one tissue section of a tissue sample from an individual with the localization and/or the expression level in the tissue section of an artificial control tissue;
   wherein a differential expression of the structure, complex or molecule is an indicator of an abnormal condition in the tissue sample of the individual.

The provision of the tissue sections in step (i) and (ii) may be carried out simultaneously or successively. For example, the artificial control tissue and the at least one tissue sample of step (ii) may be comprised in separate samples, such as separate punch samples. Alternatively, the artificial control tissue and the at least one tissue sample of step (ii) may be comprised in the same TMA or tissue block.

Steps (i) and (ii) may comprise sectioning the artificial control tissue. Alternatively, the sections may be retrieved from storage. For example, storage of paraffin sections is possible on glass or paper slides. It is, however, preferred that the sections are prepared immediately before mounting them on slides. The tissue sections may be provided for example on glass or plastic slides.

Suitable methods for detection of at least one structure, complex and/or molecule in step (iii) are well known in the art. Usually, the sections have to be pre-treated, *inter alia* to remove an embedding medium. Subsequently, the detection can be initiated. The detection can use histochemical methods, immunohistochemistry (IHC) methods, i.e. antibody-dependent, methods and/or *in situ* hybridization methods, i.e. gene probe-dependent methods.

All of the aforementioned methods are suitable for a space-resolved and semi-quantitative analysis in accordance with step (iv). Comparing of the localization and/or the expression level can be carried out manually or in a computer-assisted manner. Preferably, the process is carried out computer-assisted and automated. For example the comparing in step (iv) can be carried out using computer-assisted image analysis. Further, it is also possible to use automated data acquisition.

In a seventh aspect the invention relates to a use of an artificial control tissue of the invention or manufactured according to a method of the invention as an external control in an *in vitro* diagnostic method.

### BRIEF DESCRIPTION OF THE FIGURES

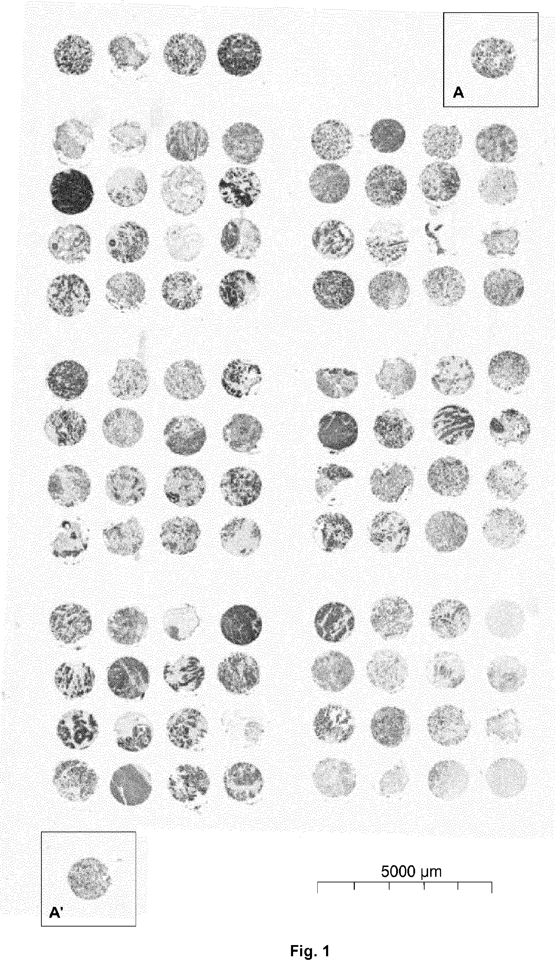

- Fig. 1: shows 100 sections from different consecutive archived tissue samples as well as two sections "A" and "A' " from the same core of the artificial control tissue A. The artificial control tissue A was generated from 100 different consecutive archived tissue samples. The punch samples ("cores") from the artificial control tissue A and the archived tissue samples had a diameter of 1 mm, were pooled into a tissue microarray and stained with hematoxylin-eosin.

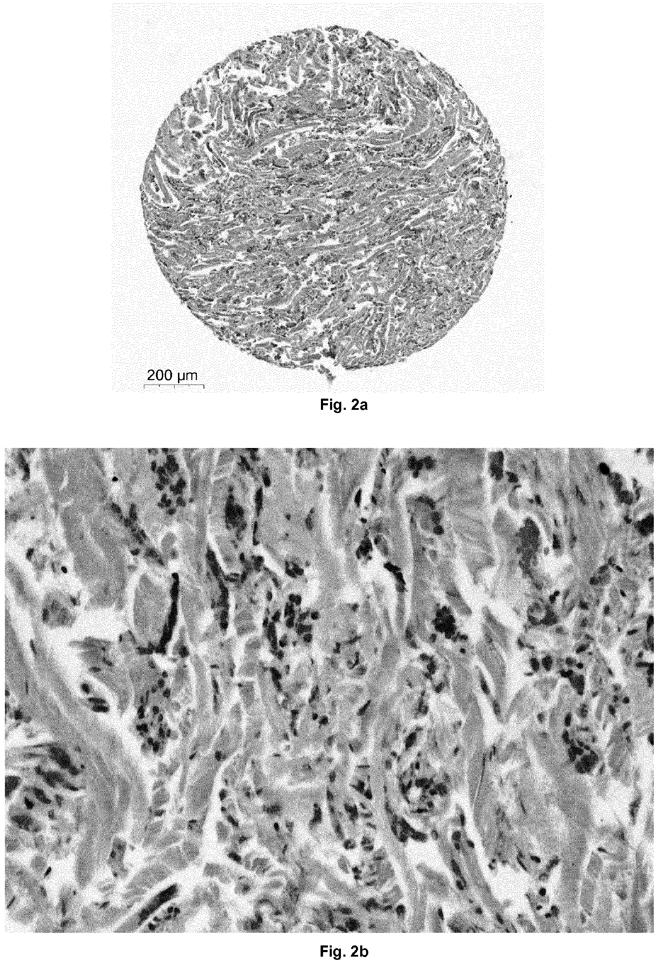

- Fig. 2: shows magnifications of the section "A" of artificial control tissue shown in Fig. 1. Fig. 2a shows an overview of the entire section; Fig. 2b shows details of the same section (original magnification x200). The magnifications show clearly the tissue fragments comprised in the artificial control tissue.

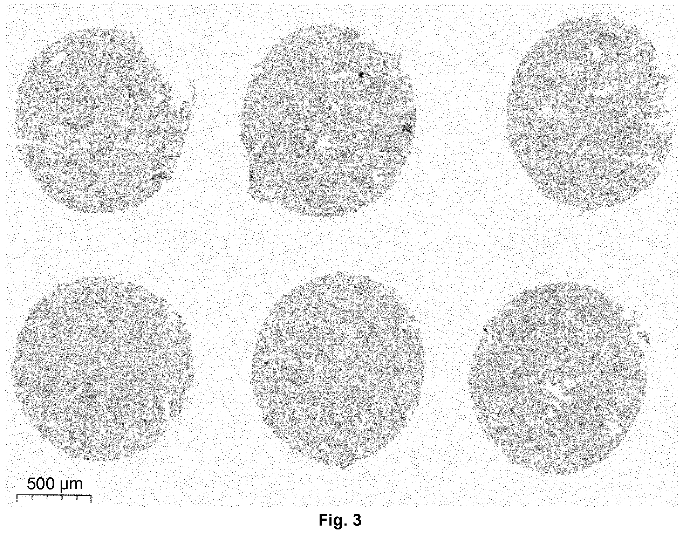

- Fig. 3: shows 6 different sections taken from 6 different punch samples ("cores") from a single preparation, i.e. from the same artificial control tissue, and reveals the homogenous mixture of cell and tissue fragments with a very low variance. The punch samples had a diameter of 1 mm and were pooled into a tissue microarray. The sections were stained with a hematoxylin-eosin stain.

### EXAMPLES

### Example 1: Manufacture of artificial control tissue

100 sample sections were taken from sequential samples of archived breast cancer biopsies. The sections had a thickness of 5 µm. The samples had previously been embedded in paraffin. The samples had a temperature of between -20°C and -80°C throughout the procedure.

All 100 sample sections were pooled into a vessel and shredded. Various shredding assemblies were tested and deemed suitable. The best results were obtained by crushing the sample sections with a mortar. It was, however, also possible to use a standard kitchen food processor. The resulting tissue fragments were of diverse size of shape and had an average diameter of about 5 to 20 µm. The obtained tissue fragments were already mixed after the shredding of the combined sections.

Subsequently, the tissue fragments were dewaxed in Xylol (10 min), then centrifuged for 5 min (1.500 rpm). 99% Ethanol was added (3 min incubation), then centrifuged for 3 min (3.000 rpm). Afterwards, these steps were repeated. To rehydrate the fragments they were passed through the descending ethanol series (2x 3 min 96%, 1x 3 min 80% Ethanol). After each of these 3 steps, the tissue was centrifuged 3 min (3.000 rpm). Samples were then washed twice in phosphate buffered saline (PBS), digested for 30 min, 37°C, 600 rpm in 0,5% Trypsin-EDTA solution and pelletized for 5 min at 7.000 rpm. Afterwards the samples were washed twice in PBS and pelletized at 14.000 rpm.

The obtained pellet was embedded in paraffin following standard protocols, resulting in artificial control tissue "A". A second artificial control tissue was produced according to the above described method using another set of archived breast cancer samples as a basis, resulting in artificial control tissue "B".

### Example 2: Manufacture of artificial control tissue enriched in Her2

The human epidermal growth factor receptor 2 (Her2) is overexpressed in certain aggressive types of breast cancer and has become an important biomarker and therapy target in a subgroup of breast cancer patients. However, the prevalence of a Her2-overexpression is low and the Her2 expression in non-overexpressing samples is regularly below the detection limit. It was therefore desirable to generate an artificial control tissue enriched in Her2 to be able to use the artificial control tissue as a positive control in Her2-dependent diagnostic.

The artificial control tissue was essentially prepared as described in Example 1. The samples were 100 consecutive Her2-positive breast cancer samples.

### Example 3: Manufacture of tissue microarrays (TMA) with artificial control tissue

6 punch samples having a diameter of 1 mm were obtained from the artificial control tissues A and B, each and pooled into two paraffin tissue microarrays TMA-A and TMA-B according to standard protocols as described e.g. by Camp et al. (2000, Laboratory Investigation, 80(12), 1943-1949) and Rimm et al. (2001, Cancer J, 7(1):24-31). Sections cut from the TMA-A were stained with a hematoxylin-eosin stain (Fig. 3). Fig. 3 shows that different punch samples from the same TMA have a highly similar and homogenous morphological appearance.

The low variance between the sections of different punch samples from the same TMA is maintained throughout the punch samples. Sections were cut from three different cutting depths of TMA-A and stained with a hematoxylin-eosin stain (data not shown). It was demonstrated that different punch samples from the same TMA have a highly similar and homogenous morphological appearance throughout their length, i.e. in different cutting depths.

Variance was also very low between sections of TMA-A and TMA-B, i.e. between separate batches of artificial control tissue. Sections were cut from TMA-A and TMA-B and stained with a hematoxylin-eosin stain (data not shown). It was seen that TMA-A and TMA-B have a highly similar morphological appearance and staining intensity.

### Example 4: Preparation of a TMA with artificial control tissue and breast cancer samples

100 punch samples having a diameter of 1 mm were obtained from the 100 consecutive, archived breast cancer samples used for generating the artificial control tissue "A". Two punch samples (A and A') having a diameter of 1 mm each were obtained from the artificial control tissue A. All punch samples were combined into a paraffin-embedded TMA. Sections cut from the TMA were stained with hematoxylin-eosin (Fig. 1a). Magnifications of one of the sections of artificial control tissue A (Fig. 2a, Fig. 2b) show the tissue fragments comprised in the artificial control tissue and the low variance within the artificial control tissue, while the original tissue samples show a high staining variability.

## Claims

1. Method for the manufacture of an artificial control tissue suitable for use as a control in histomorphological analysis comprising the steps of
(a) providing twenty-five or more tissue samples;
(b) shredding the tissue samples into tissue fragments;
(c) mixing the tissue fragments; and
(d) forming an artificial control tissue
wherein 50% or more of the tissue fragments comprised in the artificial control tissue have a maximal diameter of 500 µm or less.

2. The method of claim 1, wherein step (a) comprises providing 100 or more tissue samples.

3. The method of any of claims 1 to 2, wherein the samples provided in step (a) are tissue sections having a thickness of from 0.5 µm to 100 µm.

4. The method of any of claims 1 to 3, wherein step d) of forming an artificial control tissue comprises compacting the mixed tissue fragments, e.g. by centrifugation or other means that exert pressure on the fragments.

5. The method of any of claims 1 to 4, wherein the step d) of forming an artificial control tissue comprises embedding the mixed tissue fragments in an embedding medium, such as paraffin.

6. The method of any of claims 1 to 5, wherein the samples provided in step (a) share at least one common feature distinguishing the samples from other samples of the same type.

7. The method of any of claims 1 to 6, wherein the samples provided in step (a) express the same molecule differentially in comparison with the average localization and/or the average amount of the molecule in the same type of tissue sample.

8. Artificial control tissue suitable for use as a control in histomorphological analysis and manufactured according to the method of one of claims 1 to 7.

9. Artificial control tissue suitable for use as a control in histomorphological analysis and comprising fragments of 25 or more tissue samples,
wherein 50% or more of the tissue fragments comprised in the artificial control tissue have a maximal diameter of 500 µm or less.

10. Core or section from an artificial control tissue according to claim 8 or 9 or manufactured according to a method of any of claims 1 to 7.

11. A tissue microarray or other tissue block comprising at least one core of an artificial control tissue according to any of claims 8 to 9 or manufactured according to a method of any of claims 1 to 7.

12. Object slide comprising
- a section of an artificial control tissue according to any of claims 8 to 9 or manufactured according to a method of any of claims 1 to 7;
- a section of a core according to claim 10; or
- a section of a tissue microarray or tissue block according to claim 11 as an on-slide control.

13. *In vitro* diagnostic method comprising
(i) providing at least one tissue section of an artificial control tissue according to any of claims 8 or 9 or manufactured according to a method of any of claims 1 to 7;
(ii) providing at least one tissue section of a tissue sample from an individual;
(iii) detecting at least one structure, complex and/or molecule in the tissue samples of (a) and (b); and
(iv) comparing the localization and/or the expression level of the structure, complex and/or molecule in the at least one tissue section of a tissue sample from an individual with the localization and/or the expression level in the tissue section of an artificial control tissue;
wherein a differential expression of the structure, complex or molecule is an indicator of an abnormal condition in the tissue sample of the individual.

14. Use of an artificial control tissue according to any of claims 8 or 9 or manufactured according to a method of any of claims 1 to 7 as an external control in an *in vitro* diagnostic method.

## Patentansprüche

1. Verfahren zur Herstellung eines künstlichen Kontrollgewebes, das zur Verwendung als Kontrolle bei histomorphologischer Analyse geeignet ist, umfassend die Schritte
(a) Bereitstellen von fünfundzwanzig oder mehr Gewebeproben;
(b) Zerkleinern der Gewebeproben zu Gewebefragmenten;
(c) Mischen der Gewebefragmente; und
(d) Bilden eines künstlichen Kontrollgewebes,
wobei 50 % oder mehr der Gewebefragmente, die in dem künstlichen Kontrollgewebe enthalten sind, einen größten Durchmesser von 500 µm oder weniger aufweisen.

2. Verfahren gemäß Anspruch 1, wobei Schritt (a) Bereitstellen von 100 oder mehr Gewebeproben umfasst.

3. Verfahren gemäß einem der Ansprüche 1 bis 2, wobei die bei Schritt (a) bereitgestellten Proben Gewebeschnitte mit einer Dicke von 0,5 µm bis 100 µm sind.

4. Verfahren gemäß einem der Ansprüche 1 bis 3, wobei Schritt d) des Bildens eines künstlichen Kontrollgewebes Kompaktieren der gemischten Gewebefragmente, z. B. durch Zentrifugation oder andere Mittel, die Druck auf die Fragmente ausüben, umfasst.

5. Verfahren gemäß einem der Ansprüche 1 bis 4, wobei der Schritt d) des Bildens eines künstlichen Kontrollgewebes Einbetten der gemischten Gewebefragmente in ein Einbettmedium, wie z. B. Paraffin, umfasst.

6. Verfahren gemäß einem der Ansprüche 1 bis 5, wobei die bei Schritt (a) bereitgestellten Proben wenigstens ein gemeinsames Merkmal teilen, das die Proben von anderen Proben des gleichen Typs unterscheidet.

7. Verfahren gemäß einem der Ansprüche 1 bis 6, wobei die bei Schritt (a) bereitgestellten Proben das gleiche Molekül im Vergleich zu der der mittleren Lokalisation und/oder der mittleren Menge des Moleküls in dem gleichen Typ von Gewebeprobe unterschiedlich exprimieren.

8. Künstliches Kontrollgewebe, das zur Verwendung als Kontrolle bei histomorphologischer Analyse geeignet ist, hergestellt gemäß dem Verfahren gemäß einem der Ansprüche 1 bis 7.

9. Künstliches Kontrollgewebe, das zur Verwendung als Kontrolle bei histomorphologischer Analyse geeignet ist und Fragmente von 25 oder mehr Gewebeproben umfasst, wobei 50 % oder mehr der Gewebefragmente, die in dem künstlichen Kontrollgewebe enthalten sind, einen größten Durchmesser von 500 µm oder weniger aufweisen.

10. Kern oder Schnitt eines künstlichen Kontrollgewebes gemäß Anspruch 8 oder 9 oder hergestellt gemäß einem Verfahren gemäß einem der Ansprüche 1 bis 7.

11. Gewebe-Mikroarray oder anderer Gewebeblock, umfassend wenigstens einen Kern eines künstlichen Kontrollgewebes gemäß einem der Ansprüche 8 oder 9 oder hergestellt gemäß einem Verfahren gemäß einem der Ansprüche 1 bis 7.

12. Objektträger, umfassend
- einen Schnitt eines künstlichen Kontrollgewebes gemäß einem der Ansprüche 8 oder 9 oder hergestellt gemäß einem Verfahren gemäß einem der Ansprüche 1 bis 7;
- einen Schnitt eines Kerns gemäß Anspruch 10; oder
- einen Schnitte eines Gewebe-Mikroarrays oder Gewebeblocks gemäß Anspruch 11,
als Kontrolle auf dem Objektträger.

13. In-vitro-diagnostisches Verfahren, umfassend
(i) Bereitstellen wenigstens eines Gewebeschnitts eines künstlichen Kontrollgewebes gemäß einem der Ansprüche 8 oder 9 oder hergestellt gemäß einem Verfahren gemäß einem der Ansprüche 1 bis 7;
(ii) Bereitstellen wenigstens eines Gewebeschnitts einer Gewebeprobe von einem Individuum;
(iii) Nachweisen wenigstens einer Struktur, eines Komplexes und/oder Moleküls in den Gewebeproben von (a) und (b); und
(iv) Vergleichen der Lokalisation und/oder des Expressionsniveaus der Struktur, des Komplexes und/oder Moleküls in dem wenigstens einen Gewebeschnitt einer Gewebeprobe eines Individuums mit der Lokalisation und/oder dem Expressionsniveau in dem Gewebeschnitt eines künstlichen Kontrollgewebes;
wobei eine unterschiedliche Expression der Struktur, des Komplexes oder Moleküls ein Indikator eines anomalen Zustands in der Gewebeprobe des Individuums ist.

14. Verwendung eines künstlichen Kontrollgewebes gemäß einem der Ansprüche 8 oder 9 oder hergestellt gemäß einem Verfahren gemäß einem der Ansprüche 1 bis 7 als externe Kontrolle bei einem *in-vitro*-diagnostischen Verfahren.

## Revendications

1. Procédé destiné à la fabrication d'un tissu témoin artificiel approprié pour être utilisé comme témoin dans une analyse histo-morphologique comprenant les étapes consistant à
(a) fournir vingt-cinq échantillons tissulaires ou plus ;
(b) déchiqueter les échantillons tissulaires en fragments tissulaires ;
(c) mélanger les fragments tissulaires ; et
(d) former un tissu témoin artificiel
dans lequel 50% ou plus des fragments tissulaires compris dans le tissu témoin artificiel ont un diamètre maximal de 500 µm ou moins.

2. Procédé de la revendication 1, dans lequel l'étape (a) comprend la fourniture de 100 échantillons tissulaires ou plus.

3. Procédé de l'une quelconque des revendications 1 à 2, dans lequel les échantillons fournis dans l'étape (a) sont des coupes de tissus ayant une épaisseur de 0,5 µm à 100 µm.

4. Procédé de l'une quelconque des revendications 1 à 3, dans lequel l'étape d) de formation d'un tissu témoin artificiel comprend une compaction des fragments tissulaires mélangés, à titre d'exemple par centrifugation ou d'autres moyens qui exercent une pression sur les fragments.

5. Procédé de l'une quelconque des revendications 1 à 4, dans lequel l'étape d) de formation d'un tissu témoin artificiel comprend une inclusion des fragments tissulaires mélangés dans un milieu d'inclusion, tel que la paraffine.

6. Procédé de l'une quelconque des revendications 1 à 5, dans lequel les échantillons fournis dans l'étape (a) partagent au moins une caractéristique commune qui distingue les échantillons d'autres échantillons du même type.

7. Procédé de l'une quelconque des revendications 1 à 6, dans lequel les échantillons fournis dans l'étape (a) expriment la même molécule de manière différentielle en comparaison avec la localisation moyenne et/ou la quantité moyenne de la molécule dans le même type d'échantillons tissulaires.

8. Tissu témoin artificiel approprié pour être utilisé comme témoin dans une analyse histo-morphologique et étant fabriqué selon le procédé d'une des revendications 1 à 7.

9. Tissu témoin artificiel approprié pour être utilisé comme témoin dans une analyse histo-morphologique et comprenant des fragments de 25 échantillons tissulaires ou plus,
dans lequel 50% ou plus des fragments tissulaires compris dans le tissu témoin artificiel ont un diamètre maximal de 500 µm ou moins.

10. Partie centrale ou coupe provenant d'un tissu témoin artificiel, selon les revendications 8 ou 9, ou étant fabriqué selon un procédé de l'une quelconque des revendications 1 à 7.

11. Micro-arrangement tissulaire ou autre bloc tissulaire comprenant au moins une partie centrale d'un tissu témoin artificiel, selon l'une quelconque des revendications 8 à 9, ou étant fabriqué selon un procédé de l'une quelconque des revendications 1 à 7.

12. Lame à objet comprenant
- une coupe d'un tissu témoin artificiel, selon l'une quelconque des revendications 8 à 9, ou étant fabriqué selon un procédé de l'une quelconque des revendications 1 à 7 ;
- une coupe d'une partie centrale selon la revendication 10 ; ou
- une coupe d'un micro-arrangement tissulaire ou d'un bloc tissulaire selon la revendication 11 comme témoin de lame.

13. Procédé de diagnostic *in vitro* comprenant les étapes consistant à
(i) fournir au moins une coupe tissulaire d'un tissu témoin artificiel, selon l'une quelconque des revendications 8 ou 9, ou étant fabriqué selon un procédé de l'une quelconque des revendications 1 à 7 ;
(ii) fournir au moins une coupe tissulaire d'un échantillon tissulaire provenant d'un individu ;
(iii) détecter au moins une structure, un complexe et/ou une molécule dans les échantillons tissulaires des étapes (a) et (b) ; et
(iv) comparer la localisation et/ou le niveau d'expression de la structure, du complexe et/ou de la molécule dans l'au moins une coupe tissulaire d'un échantillon tissulaire provenant d'un individu avec la localisation et/ou le niveau d'expression dans la coupe tissulaire d'un tissu témoin artificiel ;
dans lequel une expression différentielle de la structure, du complexe ou de la molécule est un indicateur d'une condition anormale dans l'échantillon tissulaire de l'individu.

14. Utilisation d'un tissu témoin artificiel, selon l'une quelconque des revendications 8 ou 9, ou étant fabriqué selon un procédé de l'une quelconque des revendications 1 à 7, comme témoin externe dans un procédé de diagnostic *in vitro.*
